# EUROPÄISCHE PATENTSCHRIFT

(11) **EP 2 920 317 B1**
(45) Veröffentlichungstag und Bekanntmachung des Hinweises auf die Patenterteilung: **10.06.2020**
(21) Anmeldenummer: 13789296.4
(22) Anmeldetag: 08.11.2013
(51) Int. Cl.: C12P 19/14, C12P 19/02, C12P 7/24, C12P 7/18, C12P 7/14, C12P 7/40, C12P 7/46, C12P 7/50, C13K 13/00

(54) **VERFAHREN ZUR GEWINNUNG VON ZUCKERDERIVATEN**
METHOD FOR OBTAINING SUGAR DERIVATIVES
PROCÉDÉ DE PRÉPARATION DE DÉRIVÉS DU SUCRE

(30) Priorität: 14.11.2012 AT 505112012
(43) Veröffentlichungstag der Anmeldung: 23.09.2015
(73) Patentinhaber: Annikki GmbH, 8074 Raaba-Grambach (AT)
(72) Erfinder: WIRTZ, Dörthe Hendrike, 51061 Köln (DE); MAYER, Bernd, 88453 Erolzheim (DE)
(74) Vertreter: Schwarz & Partner Patentanwälte OG
(86) Internationale Anmeldenummer: PCT/EP2013/073411
(87) Internationale Veröffentlichungsnummer: WO 2014/076012

(56) Entgegenhaltungen:
- WO-A1-2006/114095
- WO-A1-2007/046417
- WO-A2-2010/124312
- US-A- 2 463 784
- US-A1- 2003 172 850
- NOVICK N J ET AL: "L-arabinose metabolism in Azospirillum brasiliense", JOURNAL OF BACTERIOLOGY, AMERICAN SOCIETY FOR MICROBIOLOGY, WASHINGTON, DC; US, Bd. 149, Nr. 1, 1. Januar 1982 (1982-01-01), Seiten 364-367, XP003012015, ISSN: 0021-9193

## Beschreibung

Die vorliegende Erfindung betrifft ein Verfahren zur Gewinnung von Arabonsäure aus einem Hemicellulose-enthaltenden Material.

In den letzten Jahren findet betreffend nachwachsende Rohstoffe ein Umdenken statt. Es wird versucht anstelle von fossilem Rohstoff auf den nachwachsenden Rohstoff als Quelle für Energie und chemische Produkte umzusteigen. Nachwachsender Rohstoff (Nawaro) schließt einen land- und forstwirtschaftlicher Rohstoff pflanzlichen oder tierischen Ursprungs ein, der nicht als Nahrungs- oder Futtermittel eingesetzt wird. Er kann stofflich, aber auch energetisch genutzt werden. Nawaros haben viele Vorteile wie die Schonung der nur begrenzt vorhandenen fossilen Rohstoffe, eine ausreichende Verfügbarkeit und die Erschließung neuer Märkte für die Überproduktion bei der Landwirtschaft.

Auch Lignocellulose gewinnt als Nawaro an Bedeutung (Kamm and Kamm 2004, Appl Microbiol Biotechnol. 64(2): 137-45). Sie besteht aus 3 verschiedenen chemischen Hauptfraktionen: Cellulose, ein C6 Polymer aus Glucoseeinheiten; Hemicellulose, bestehend aus verschiedenen C5 Zuckern, wie z.B. Xylose; und Lignin als ein Phenol-Polymer. Eine Möglichkeit die Lignocellulose zu nutzen ist das Vergasen, sodass die sogenannte "syngas platform" erhalten wird. Der Rohstoff wird unter limitierter Sauerstoffzugabe verbrannt um ein Sythesegas reich an CO₂, CO, H₂, CH₄ und N₂ sowie Teer zu produzieren (Bridgwater, 2003). Das Synthesegas kann dann wiederum genutzt werden, um Kraftstoff und Chemikalien zu produzieren, z.B. mittels Fischer-Tropsch-Synthese (Tijmensen *et al.,* 2002). Eine zweite Möglichkeit ist die sogenannte "sugar platform". Hier wird die Lignocellulose zunächst in die 3 Hauptbestandteile zerlegt und diese werden dann weiter in Produkte umgesetzt. Die Xylose kann z.B. zu Xylitol oder aber Furfural umgesetzt werden. Die Glucose kann zur Fermentation eingesetzt werden oder in Hydroxymethylfurfural (HMF) überführt werden. Häufig wird das Lignin zur Energieerzeugung genutzt und einfach nur verbrannt (Saake and Lehnen, 2007, Ullmann's Encyclopedia of Industrial Chemistry. Wiley-VCH Verlag GmbH & Co*).*

Die Zucker liegen in der Lignocellulose in eng vernetzten, polymeren Strukturen in Form teilweise kristalliner Cellulose und amorpher, die Cellulose umhüllender Hemicellulosen vor. Im Zuge der Zellwandsynthese werden die Hohlräume mit Lignin ausgefüllt, wodurch sich ein äußerst dichter Komplex ergibt. Die Dichte der Strukturen macht Enzymen, wie z.B. Cellulasen oder Hemicellulasen eine Zugänglichkeit unmöglich, sie sind durch ihr relativ hohes Molekulargewicht nicht imstande in die Poren zu gelangen (Himmel et al., 2007, Science. 315 (5813): 804-7). Daher ist es notwendig, dass vor der enzymatischen Behandlung ein chemischer Schritt erfolgt, der die Porosität der Lignocellulose erhöht. Dieser Schritt wird als Pretreatment (Aufschluss) bezeichnet. Im Aufschluss wird die polymere Lignocellulosematrix aufgebrochen, und die Cellulosefasern werden somit exponiert, sodass sie für Enzyme zugänglich werden. Der Aufschluss ist ein kritischer Schritt, der als einer der kostenaufwendigsten Schritte der Biorefinery beschrieben wird (Mosier et al., 2005, Bioresour Technol. 96(6):673-86). Zum anderen hat er auch einen sehr großen Einfluss auf die folgenden Schritte wie Hydrolyse, Fermentation, downstream Prozesse und auch den Abfall der Prozesse (Alvira et al., Bioresour Technol. 101 (13): 4851-61).

Die gängigen Verfahren des Aufschluss zielen entweder darauf ab, primär die Hemicellulosen zu verflüssigen (z.B. steam explosion-, dilute acid-pretreatment) oder die Erhöhung der Porosität durch Verflüssigung von Lignin (z.B. lime-, ammonia-pretreatment) zu erreichen. Diese Verfahren haben einen gravierenden Nachteil: Sie sind entweder energieaufwändig und laufen vorwiegend bei Temperaturen knapp unter 200°C ab. Oder aber sie erfordern eine aufwändige Rückgewinnung der Aufschlusschemikalien. Die Art der Vorbehandlung kann einen starken Einfluss auf die Enzymaktivität und die Ausbeute bei anschließenden biokatalytischen Prozessen haben. Bei hohen Reaktionstemperaturen entstehen vielfach toxische Abbauprodukte (z.B. Furfural) welche im Falle einer unmittelbar angeschlossenen Ethanol-Gärung die Hefen hemmen können (Chandra et al., 2007, Adv Biochem Eng Biotechnol.108: 67-93*;* Mansfield et al., 1999, Biotechnol Prog. 15 (5): 804-816).

Als Hemicellulose ist Xylan ein nicht homogenes Polymer. Hemicellulosen beinhalten hauptsächlich Pentosen (C5), wie D-Xylose und L-Arabinose aber auch Hexosen (C6) wie D-Glucose, D-Mannose und D-Galactose und auch Zuckersäuren wie Glucuronsäure und 4-O-methyl-D-Glucuronsäure. Hemicellulosen haben üblicherweise einen Polymerisationsgrad kleiner 200 (Jørgensen *et al.,* 2007). Hemicellulosen werden nach den Zuckern benannt, aus denen sie aufgebaut sind, z.B. das im Weizenstroh enthaltene Arabinoglucuronoxylan, das aus dem Xyloserückgrat besteht und Seitenketten aus Arabinose und Glucuronsäure enthält. Die Xyloseeinheiten können noch mit Acetat und Ferula- bzw. Coumarsäure verestert sein (Polizeli et al., 2005, Appl Microbiol Biotechnol. 67(5): 577-91). Für den enzymatischen Abbau des Xylans sind Endo-Xylanasen, β-Xylosidasen, α-Glucuronidasen, α-L-Arabinofuranidasen und Esterasen notwendig (Polizeli et al., 2005, ibidem). Die Endo-Xylanase spaltet die glycosidische Bindung im Xylan-Rückgrat und reduziert damit den Grad der Polymerisierung des Substrates. Das Hauptprodukt der Hydrolyse sind β-D-Xylopyranosyl-Oligomere, aber auch kleine Mengen an Mono- Di- und Trisacchariden. β-Xylosidasen spalten die Xylooligosaccharide in monomere Xylose. Die restlichen Enzyme weisen Aktivität gegenüber den Seitenketten auf und trennen diese ab. α-Glucuronidasen spalten die Glucoronsäurereste vom Rückgrat ab, α-L-Arabinofuranidasen die Arabinoseseitenketten. Eine Verfahren unter Einsatz mehrerer α-L-Arabinofuranidasen wird in WO 2006/114095 zur Freisetzung von Xylose und Arabinose offenbart. Esterasen spalten die Esterbindungen des Xylans zu Seitenketten wie Acetat oder p-Coumarsäure bzw. Ferulasäure (Collins et al., 2005, FEMS Microbiol Rev. 29(1): 3-23). Die Abspaltung der Seitenketten ist essentiell dafür, dass Endo-Xylanase und β-Xylosidase das Xylan vollständig zerlegen können.

### Stand der Technik

Die Xylanhydrolyse dient der Spaltung des Zuckerpolymers in Zuckeroligomere oder Zuckermonomere. Hier können verschiedene Ziele unterschieden werden. In einigen Anwendungen ist es sinnvoll, beide Zuckerpolymere, Cellulose und Xylose, in Monomere zu spalten. Insbesondere bei der Herstellung von fermentierbaren Zuckern aus Biomassen ist dies der Fall. In anderen Anwendungen soll jedoch die Cellulose als Polymer erhalten bleiben, das Xylan aber zu Oligomeren oder Monomeren gespalten werden. Die Xylanhydrolyse kann chemisch oder enzymatisch erfolgen. Weiterhin kann die Xylanhydrolyse gleichzeitig mit der Abtrennung von lignocellulosischem Material erfolgen, oder in einem separaten Schritt.

In US 3,523,911 wird eine chemische Methode beschrieben, bei der die Biomasse bei Temperaturen von 100-150°C mit säurehaltigen Dampf bearbeitet wird, der dann bei der Kondensation aus dem Material Zucker herauslöst. Bei dieser Methode wird jedoch sehr viel Säure verbraucht und es wird ein Hydrolysat mit einer nur sehr geringen Xylosekonzentration erhalten. Wenn z.B. 15 % Xylan bezogen auf die Trockenmasse aus Bagasse hydrolysiert wird, dann enthält das Hydrolysat nur 3 % Xylose, was auf die hohe Wassermenge, die während des Prozesses absorbiert wird, zurückzuführen ist. Der hohe Säureverbrauch und die Kosten für das Aufkonzentrieren der Zuckerlösung machen den Prozess unrentabel.

In US 7,932,063 wird eine Methode beschrieben, bei der die Biomasse zunächst mit einer wässrigen, Ammoniak-enthaltenden Lösung aufgeschlossen wird. Das erhaltene Produkt wird dann mit einem "saccharification enzyme", einem Zucker-spaltenden Enzym, umgesetzt um fermentierbare Zucker zu erhalten. Das Enzym kann mehrere Aktivitäten wie Glycosidase, Peptidase, Lipase, Ligninase und Esterase enthalten. Dadurch wird gewährleistet, dass möglichst alles an vorhandenem Zuckerpolymer in Monomere zerlegt wird. So wird eine hohe Zuckerausbeute bei hoher Zuckerkonzentration des Hydrolysats erzielt. Der Nachteil ist die Unspezifität der Hydrolyse. Bei dieser Methode besteht nicht die Möglichkeit nur das Xylan in einem lignocellulosischem Material zu spalten und die Cellulose als Polymer zu erhalten.

In AT 509 307 A1 wird eine Methode beschrieben bei der Biomasse, die über eine alkalische Alkohollösung mit Temperaturen unter 100°C aufgeschlossen wurde, mit einem Kohlenhydrat-spaltenden Enzym behandelt wird, um Zuckermonomere zu erhalten. Wird hier eine reine Xylanase als Enzym eingesetzt, so wird nur das Xylan zerlegt, die Cellulose bleibt als Polymer erhalten. Die aus dem Xylan erhaltene Xylose kann dann mit einer Xylosereduktase zu Xylitol umgesetzt werden, ohne dass es einer Abtrennung der Xylose aus dem Hydrolysat bedarf. So werden aus einer vorbehandelten, Hemicellulose-haltigen Biomasse C5 Zucker in hoher Konzentration erhalten. Ein Nachteil des Verfahrens ist jedoch, dass die C5 Zucker aus der Hydrolyse, oder aber die reduzierten Folgeprodukte, nur aufwendig aus der Reaktionslösung abgetrennt werden können. In der Lösung befinden sich noch Reste an löslichen Xylan, Xylooligosaccharide und Enzyme bzw. Proteine. Um Xylose oder Xylitol aus dieser Lösung zu isolieren, ist eine Kristallisation notwendig. Ein vergleichbares Verfahren wird in WO 2010/124312 offenbart.

In der Literatur werden Methoden zur Kristallisation von Xylitol aus Hydrolysaten aufgrund der niedrigen Konzentration als schwierig beschrieben (Wei et al., 2010; Frontiers of Chemical Engineering in China 4(1): 57-64 ; Rivas et al., 2006, Agric Food Chem. 54(12): 4430-5). Außerdem muss, auf Grund der komplexen Zusammensetzung solcher Hydrolysate auch noch ein Reinigungsschritt vor der Kristallisation erfolgen. Rivas *et al.* (2006) beschreiben eine Methode, bei der ein Reinigungsschritt mit Aktivkohle erfolgt und dann die Konzentration des Xylitols durch Verdampfen des Lösemittels erreicht wird. Wei *et al.* verwenden einen Reinigungsschritt mit Aktivkohle und Ionenaustauscher bevor die Konzentration des Xylitols durch Verdampfen des Lösemittels erhöht wird. In beiden Fällen erfolgt die Kristallisation durch Zugabe von Ethanol in der Kälte. Vor allem die Aufkonzentrierung der Xylitollösung durch Verdampfen des Lösemittels ist sehr kostenintensiv und nicht optimal für einen großtechnischen Prozess.

US 2,463,784 offenbart die Herstellung von Zuckersäuren aus Aldopentosen in Kulturen von Bakterien der Gattung *Pseudomonas.*

Von Watanabe *et al.* wird ein Arabinose-Stoffwechselweg in Mikroorganismen beschrieben, bei dem Arabinose Phorphorylierungs-unabhängig in 5 Schritten zu α-Ketogluterat umgesetzt wird (Watanabe et al., 2005; Nucleic Acids Symp Ser (Oxf). (49):309-10 ; Watanabe et al. J Biol Chem. 281(44):33521-36, 2006, WO2007/046417). Der Zucker wird zunächst durch eine L-Arabinose-1-Dehydrogenase zum L-Arabino-γ-lacton oxidiert. Das Enzym überträgt bei der Reaktion die Elektronen aus dem Substrat auf NADP⁺ bzw. NAD⁺. Das L-Arabino-γ-lacton wird durch die L-Arabinolactonase zum L-Arabonat geöffnet. Das Enzym braucht keinen Kofaktor. Am Arabonat folgen zwei Dehydratisierungsschritte. Der erste wird durch die L-Arabonat-Dehydratase katalysiert, die das L-Arabonat in L-2-Keto-3-deoxyarabonat (L-KDA) überführt. Die L-KDA-Dehydratase setzt dieses dann zu α-Ketoglutarsäure-Semialdehyd (a-KGS) um. Auch die beiden Dehydratasen katalysieren die Reaktionen ohne löslichen Kofaktor (Watanabe et al., 2006, J Biol Chem. 281(39):28876-88). Zum Schluss wird der Semialdehyd dann durch die α-KGS-Dehydrogenase zum α-Ketoglutarat oxidiert. Dieses Enzym braucht wiederum NAD⁺ bzw. NADP⁺ zur Katalyse der Oxidation (Watanabe et al., 2006, ibidem). Diese Umsetzung eignet sich so jedoch nicht für eine großtechnische Anwendung, da zwei der fünf Schritte die Kofaktoren NAD(P)⁺ in stöchiometrischen Mengen benötigen, was sehr hohe Kosten verursachen würde. Auch Novick und Jyler beschreiben einen enzymkatalysierten Arabinose-Stoffwechselweg in einem Mikroorganismus (Novick et al. 1982, Journal of Bacteriology, 149(1): 364-7).

In US 2006/0234363 A1 wird der von Watanabe *et al.* (2006) beschriebene Arabinose-Stoffwechselweg zum Teil genutzt, um aus Arabinose und Xylose 1,2,4-Butantriol in Mikroorganismen herzustellen. Hier wird zunächst der Zucker mittels Dehydrogenase und Lactonase zum Lacton oxidiert und zur entsprechenden Säure hydrolysiert. Dann erfolgt in den Zellen eine Dehydratisierung an C3, so dass D- bzw. L-3-Deoxy-glycero-pentulosonic acid entsteht, je nachdem, welcher Zucker eingesetzt wird. Im Anschluss erfolgt dann jedoch eine Decarboxylierung der Säuregruppe, so dass D- bzw. L-Dihydroxybutanal entsteht. Danach wird noch das Aldehyd an C1 reduziert, so dass D- bzw. L-1,2,4-Butantriol entsteht. Da bei dieser Anwendung in ganzen Zellen gearbeitet wird, ist die Zugabe der stöchiometrischen Mengen an Kofaktor für die Redoxschritte nicht notwendig. Der Nachteil ist jedoch, dass in Zellen generell mit geringeren Substratkonzentrationen gearbeitet werden kann als in einem zellfreien System. Hinzu kommt, dass bei Arbeiten in ganzen Zellen das System nicht so effizient ist, da ein Teil der Zucker, die eingesetzt werden den Organismen zum Überleben dienen und so nicht in Produkt umgesetzt werden.

In US 6,284,904 wird eine Methode beschrieben, die der Entfernung organischer Säuren, wie zum Beispiel Succinat, aus industriellen Lösungen wir Fermentationsansätzen oder Hydrolysaten dient. Hierbei wird die Lösung über einen Anionen-Austauscher gegeben und dieser unter Bedingungen gewaschen bei denen die organischen Säuren nicht eluiert werden. Im Anschluss werden die organischen Säuren durch Zugabe stärkerer, anorganischer Anionen eluiert. So können organische Anionen aus einer komplexen Lösung, wie z.B. einem Hydrolysat, isoliert und auch aufkonzentriert werden.

In US 6,187,570 wird eine Methode beschrieben, durch die Derivate der Gluconsäure mittels Elektrodialyse aus einem Fermentationsansatz oder einem zellfreien, biokatalytischen Ansatz isoliert werden können. Zwischen Kathode und Anode werden abwechselnd mehrere Anionen- und Kationen-Membranen angebracht. Der Block aus Kathode, Anode und den Zwischenmembranen ist mit einem Elektrolyten gefüllt. Es gibt "Feed-Kompartimente", in die die Lösung, Fermentationsansatz oder zellfreier, biokatalytischer Ansatz eingebracht werden. Außerdem gibt es "Konzentrations-Kompartimente" in denen die Säuren aufkonzentriert werden. Beide Kompartimente sind mit einer Anionen- und einer Kationen-Membran voneinander getrennt. Wenn eine Spannung angelegt wird, so wandert die negativ geladene Säure durch die Anionen-Membran in das Konzentrations-Kompartiment, während die ungeladenen Bestandteile der Lösung im Feed-Kompartiment verbleiben. So wird die Gluconsäure bzw. die Derivate dieser Säure von neutralen Bestandteilen abgetrennt und aufkonzentriert.

In US 5,464,514 wird eine Methode beschrieben, bei der Zucker über ihre unterschiedliche Tendenz an eine schwache Säure zu binden, getrennt werden. Die Trennung erfolgt dann über Elektrodialyse. Als schwache Säure wird Borsäure gewählt. Unterschiedliche Zucker weisen eine unterschiedliche Tendenz auf, an Borsäure zu binden. Die Verbindung aus Zucker und Borsäure ist negativ geladen und wandert im elektrischen Feld, während die Zucker, die nicht an die Borsäure binden ungeladen bleiben. Die Elektrodialysezelle besteht aus Kathode und Anode zwischen die zwei Kationen-Austauscher-Membranen angebracht wurden. Zwischen den beiden Kationen-Austauscher-Membranen befindet sich eine Anionen-Austauscher-Membran, die den Zwischenraum in 2 Kompartimente teilt. Kompartiment I befindet sich auf Seiten der Kathode und Kompartiment II auf Seiten der Anode. Wird nun eine Lösung der zu trennenden Zucker in Kompartiment I eingebracht und eine Spannung angelegt, wandern die negativ geladenen Ionen, das heißt die Zucker, die an die Borsäure gebunden haben, durch die Anionen-Austauscher-Membran in Kompartiment II. Die ungeladenen Zucker verweilen in Kompartiment I. Das Verfahren wurde für die Trennung von Lactose und Lactulose sowie für die Trennung von Glucose und Fructose getestet. Diese Methode beruht jedoch darauf, dass zwischen den Zuckern eine unterschiedliche Affinität der Bindung an Borsäure besteht. Aber auch ein Teil der Zucker mit geringerer Affinität für die Borsäure wird die Säure binden und damit ins Kompartiment II übergehen. Somit ist keine Trennung der Zucker möglich, lediglich ändert sich das Verhältnis der Zucker zueinander. Im Falle von Fructose und Glucose war das Verhältnis der Transferraten 1,4 zu 1. Die Separation kann durch mehrere Elektrodialyseschritte erhöht werden. Borsäure und Zucker können anschließend in einem weiteren Elektrodialyse-Schritt voneinander getrennt werden. Der Nachteil der Methode ist, dass eine gute Separation nur durch viele Elektrodialyse-Schritte erzeugt werden kann. Außerdem wird bei dieser Methode nicht zwischen monomeren und dimeren bzw. oligomeren Zuckern unterschieden. Eine gute Trennung kann nur erzeugt werden, wenn die zu trennenden Substanzen sich stark in der Bindungstendenz an die Borsäure unterscheiden. Weiterhin von Nachteil ist, dass mit Borsäure eine zusätzliche (toxische) Komponente eingesetzt werden muss.

In der US 2003/0172850 ist eine Zusammensetzung beschrieben, die als Zusatz für Zementmischungen dient, und die
(A) eine Lignosulfonsäure oder Salze davon; eine Aldohexonsäure oder Salze davon; eine Hexuronsäure oder Salze davon, "hexaric acids" oder Salze davon; oder Mischungen davon; und
(B) zumindest eine Aldopentonsäure oder Salze davon enthält.

### Beschreibung der Erfindung

Es wurde nun ein Verfahren gefunden, das eine direkte Nutzung von Zuckern erlaubt, die bei der Hydrolyse von lignocellulosehaltigem (bzw. hemicellulosehaltigem) Materials entstehen.

In einem Aspekt stellt die vorliegende Erfindung ein Verfahren zur Gewinnung von Arabonsäure aus hemicellulosehaltigem Material zur Verfügung, wobei
a) das hemicellulosehaltige Material enzymatisch oder nicht-enzymatisch hydrolysiert wird und das erhaltene Hydrolysat als freigesetzte Zucker Arabinose und Xylose enthält, und
b) das erhaltene Hydrolysat einer Umsetzung unterworfen wird, gekennzeichnet durch die Kombination der folgenden Maßgaben:
   - die Arabinose wird mit Hilfe einer Oxidoreduktase in das γ-Arabinolacton übergeführt und γ-Arabinolacton zu Arabonsäure hydrolysiert, wobei von der Oxidoreduktase Redoxkofaktoren zu NADH und/oder NADPH reduziert werden, und
   - die reduzierten Redoxkofaktoren NADH und/oder NADPH werden im selben Reaktionsansatz mittels einer Alkoholdehydrogenase, einer Lactat-Dehydrogenase, einer Xylose-Reduktase, einer Oxidase, und/oder eines oder mehrerer Redoxenzyme, die an eine Elektrode gekoppelt werden, in den oxidierten Zustand NAD⁺ und/oder NADP⁺ übergeführt.

Ein Verfahren, das durch die vorliegende Erfindung zur Verfügung gestellt wird, wird hierin auch als "Verfahren nach (gemäß) vorliegender Erfindung" bezeichnet.

In einem Verfahren gemäß vorliegender Erfindung können sowohl die Hydrolyse des hemicellulosehaltigen Materials, als auch die Überführung freigesetzter Zucker in Arabonsäure, als eine Verbindung die zumindest eine ionische Bindungsstelle aufweist, in einem Reaktionsansatz stattfinden. Das bedeutet, dass das Hydrolysat vor der Überführung freigesetzter Zucker in Arabonsäure, nicht isoliert werden muss (Eintopfreaktion).

Hemicellulosehaltiges Material, das in einem Verfahren gemäß vorliegender Erfindung verwendet werden kann, ist aus lignocellulosischem Material erhältlich, z.B. durch Vorbehandlung von lignocellulosehaltigem Material.

In einem Verfahren gemäß vorliegender Erfindung schließt "lignocellulosehaltiges Material" insbesondere lignocellulosehaltige Biomasse ein, z.B. Einjahrespflanzen, wie (trockene) Gräser, oder Teile von Gräsern, vorzugsweise Gräser, Stroh, Energiegräser, wie z.B. Switchgrass, Elefantengras oder Abaca, Sisal, Bagasse, oder untypische Lignocellulosesubstrate, wie Spelzen, z.B. Deckspelzen, wie Reisspelzen, besonders bevorzugt Stroh, Energiegräser, Bagasse oder Spelzen, noch mehr bevorzugt Stroh oder Bagasse.

Lignocellulosehaltige Biomasse zum Einsatz in einem Verfahren gemäß der vorliegenden Erfindung wird bevorzugt vorbehandelt, z.B. durch Behandlung mit einer alkalischen, wässrigen Alkohollösung, bevorzugt bei Temperaturen von 50 bis 100°C, z.B. 100 °C und darunter, vorzugsweise 85°C und darunter, besonders bevorzugt 71°C, behandelt. Bevorzugt beträgt dabei der Feststoffgehalt des lignocellulosischen Materials in der wässrigen Lösung 1-40 Gew-%, beispielsweise 3-30 Gew-% Lösung und der Feststoff liegt vorzugsweise in einer Stoffdichte von 1-40 Gew-%, z.B. 3-30 Gew-%, insbesondere 5-20 Gew-% vor.
Als Alkohol bei der Vorbehandlung wird bevorzugt ein aliphatischer Alkohol, wie ein C₁₋₆-Alkohol, besonders bevorzugt ein C₁₋₄-Alkohol, wie Ethanol oder Isopropanol eingesetzt. Der pH-Wert der alkoholischen Lösung, der vorzugsweise 10 bis 14 beträgt, kann mit einer Base, bevorzugt einer anorganischen Base, beispielsweise einem Hydroxid, wie Natronlauge, Kalilauge eingestellt werden. Die Basenkonzentration beträgt bei der Reaktion typischerweise von 1 bis 10 mol L⁻¹, vorzugsweise von 2 bis 6 mol L⁻¹, noch mehr bevorzugt von 4,5 bis 5,5 mol L⁻¹. Diese besondere Ausführungsform der Vorbehandlung von lignocellulosehaltigem Material, das in einem Verfahren nach vorliegender Erfindung bevorzugt eingesetzt wird, beruht auf der Erkenntnis, dass sich ein mit einer wässrigen basischen Lösung, welche einen Alkohol, insbesondere einen C₁₋₆-Alkohol und einen pH-Wert von 10,0 bis 14,0 aufweist, behandeltes Material, das mit Cellulose und Hemicellulose angereichert ist, als besser verwertbares Material für einen enzymatischen Abbau zu Kohlenhydratspaltprodukten eignet, als Material, das gemäß einer anderen Ausführungsform vorbehandelt wird.

Das lignocellulosehaltige, bzw. hemicellulosehaltige Material, das in einem Verfahren gemäß vorliegender Erfindung eingesetzt wird, wird einer enzymatischen, oder nichtenzymatischen, bevorzugt einer enzymatischen Hydrolyse unterworfen. Eine nicht-enzymatische Hydrolyse zum Erhalt eines zuckerhaltigen Hydrolysats kann nach üblichen Methoden durchgeführt werden, z.B. durch eine säurekatalysierte Hydrolyse. Für die enzymatische Hydrolyse, die gemäß bekannten Verfahren erfolgen kann, werden geeignete Enzyme verwendet, beispielsweise Endo-Xylanasen, β-Xylosidasen, α-Arabinofuranosidasen, Glucuronidasen, Cellulasen und Mischungen aus solchen Enzymen.

In einem Verfahren gemäß vorliegender Erfindung wird das Hemicellulose-enthaltende Material, das beispielsweise nach einer wie oben beschriebenen Vorbehandlung erhalten wird, vorzugsweise in einer wässrigen Lösung in einer Stoffdichte von 1-40 Gew-% Trockenmasse eingesetzt.

Verbindungen, die zumindest eine ionische Bindungsstelle aufweisen, schließen Verbindungen ein, die zumindestens eine Bindungsstelle aufweisen, die zur Salzbildung geeignet ist, wie z.B. Säuregruppen der Formel -(COO⁻)ₙRⁿ⁺, worin R Wasserstoff oder ein Kation, wie z.B. ein Alkali oder Erdalkalikation, z.B. Na⁺, K⁺, Ca⁺⁺ bedeutet und n die Ladung, die das Kation aufweist und die von dessen Wertigkeit abhängt, bedeutet. Freigesetzte Zucker in einem Verfahren gemäß vorliegender Erfindung sind Arabinose, z.B. L-Arabinose und Xylose, z.B. D-Xylose.

Die Überführung des Zuckers Arabinose in die Form einer Verbindung, die zumindestens eine ionische Bindungsstelle aufweist, d.h. Arabonsäure erfolgt enzymatisch. Sie kann beispielsweise nach dem folgenden Reaktionschema 1 erfolgen, worin die Überführung von Arabinose in alpha-Ketoglutarsäure über enzymatische Oxidation und Hydrolyse zur Arabonsäure gezeigt ist, also die Überführung eines Zuckers in eine Verbindung, die eine Säuregruppe aufweist. Durch Zugabe entsprechender Kationen, beispielsweise in der Form von Hydroxiden, wie NaOH, KOH, Ca(OH)₂ kann die Säuregruppe, falls gewünscht in ein Salz übergeführt werden. R⁺ im Reaktionsschema 1 bedeutet Wasserstoff oder, wie im gezeigten Fall, ein einwertiges Kation, wie Na⁺ oder K⁺.

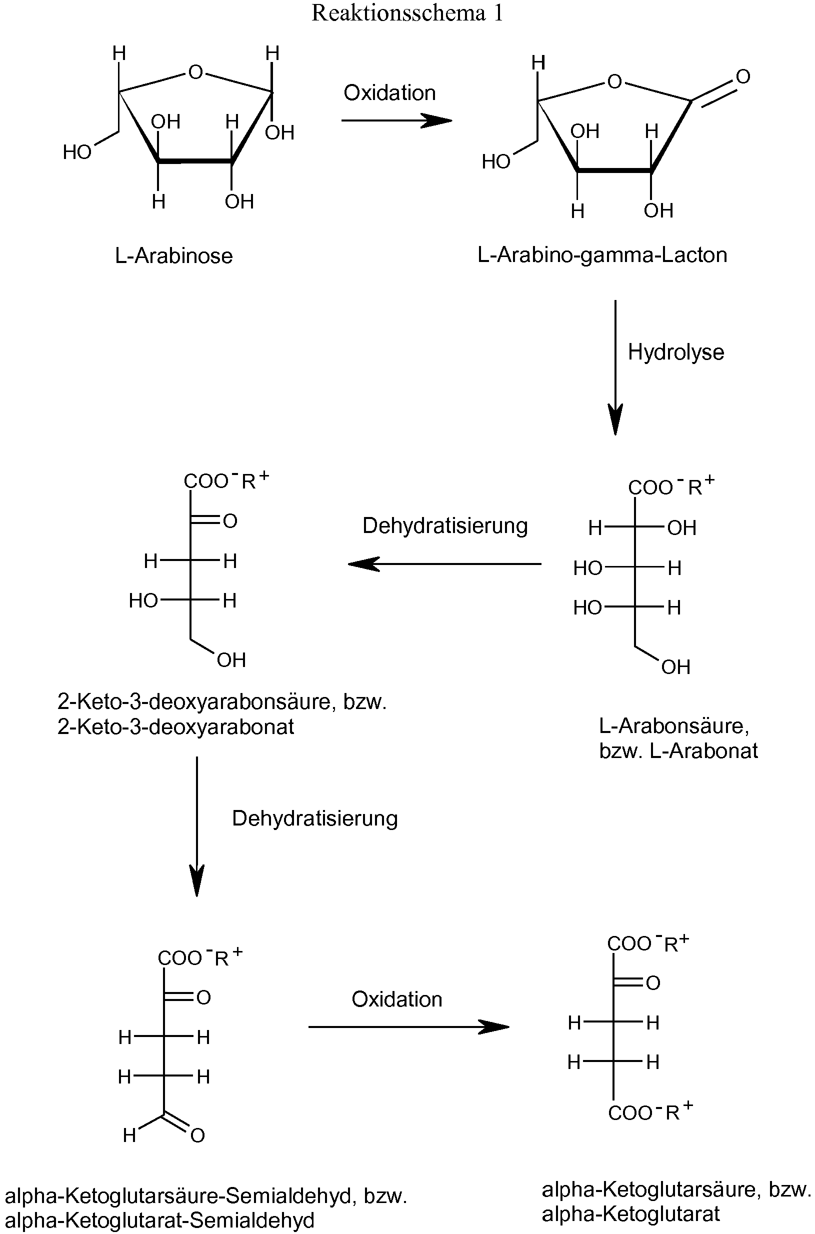

Es ist ein Vorteil in einem Verfahren gemäß vorliegender Erfindung, dass Arabinose als ein durch die Hydrolyse freigesetzter Zucker durch die enzymatische Behandlung in der Form einer Verbindung vorliegt, die zumindestens eine ionische Bindungsstelle aufweist, durch Anwendung weiterer spezifischer Enzyme direkt im Hydrolysat zu gewünschten Endprodukten umgewandelt werden kann. Dies ist im oben gezeigten Reaktionsschema am Beispiel der Umwandlung der Arabonsäure in alpha-Ketoglutarsäure, bzw. im Falle dass es als Salz voliegt, alpha-Ketoglutarat, das ein wertvolles Produkt in der organischen Chemie darstellt, gezeigt. Dazu können im gezeigten Fall Enzyme, die spezifische Dehydratisierungsreaktionen an der Arabonsäure bzw. deren Folgeprodukten katalysieren, eingesetzt werden.

Im Verfahren gemäß vorliegender Erfindung wird der freigesetzte Zucker Arabinose mit Hilfe einer Oxidoreduktase in ein Lacton, d.h. Arabino-γ-Lacton, übergeführt und dieses in die entsprechende Carbonsäure, d.h. Arabonsäure, hydrolysiert, insbesondere durch enzymatische, nicht-enzymatische und/oder spontane Hydrolyse.

Zum Erhalt spezifischer, gewünschter Verbindung, kann die erhaltene Arabonsäure, anschließend z.B. mittels einer Dehydratase, z.B. mit Hilfe von L-Arabonat-Dehydratase an einer gewünschten Position, bei Arabinose beispielsweise in Position C3, dehydratisiert werden, sodass eine entsprechende Ketocarbonsäure, d.h. (L-)2-Keto-3-deoxyarabonsäure, entsteht. Die erhaltene Ketocarbonsäure kann, falls gewünscht weiter dehydratisiert werden, beispielsweise unter Verwendung einer Dehydratase, z.B. bei Arabonsäure in Position C4, sodass ein Ketocarbonsäure-Semialdehyd, z.B. im Fall der (L-)2-Keto-3-deoxyarabonsäure unter Verwendung der L-2-Keto-3-deoxyarabonat-Dehydratase der α-Ketoglutarsäure-Semialdehyd gebildet wird. Der erhaltene Ketocarbonsäure-Semialdehyd kann, falls gewünscht, beispielsweise mit Hilfe einer Oxidoreduktase, zum Erhalt einer Dicarbonsäure oxidiert werden; im Falle eines α-Ketoglutarsäure-Semialdehyds z.B. mit Hilfe einer α-Ketoglutarat-Semialdehyd-Dehydrogenase zu Erhalt der α-Ketoglutarsäure.

In einem weiteren Aspekt stellt die vorliegende Erfindung ein Verfahren gemäß vorliegender Erfindung zur Verfügung, das dadurch gekennzeichnet ist, dass eine Arabonsäure, die gemäß der vorliegenden Erfindung erhalten wurde, zu 2-Keto-3-deoxyarabonsäure dehydratisiert wird, z.B. mittels einer Dehydratase, und,
in einem weiteren Aspekt, dass 2-Keto-3-deoxyarabonsäure, die gemäß der vorliegenden Erfindung erhalten wurde, z.B. mittels einer Dehydratase, zuem Ketocarbonsäure-Semialdehyd α-Ketoglutarsäure-Semialdehyd weiter dehydratisiert wird, und,
in einem weiteren Aspekt, dass der α-Ketoglutarsäure-Semialdehyd z.B. mittels einer Oxidoreduktase, zur Dicarbonsäure α-Ketoglutarsäure oxidiert wird.

Unter einem "Ketocarbonsäure-Semialdehyd", insbesondere α-Ketoglutarsäure-Semialdehyd, ist bei einem Verfahren gemäß vorliegender Erfindung eine aliphatische Verbindung, bei der ein endständiges C-Atom als Carboxylgruppe, ein anderes endständiges C-Atom als Formylgruppe, sowie eines der übrigen C-Atome als Ketogruppe vorliegt, zu verstehen.

Der/Die von einer oder mehreren Oxidoreduktasen reduzierte(n) Redoxkofaktor(en) NADH und/oder NADPH wird/werden im selben Reaktionsansatz mittels mindestens einer weiteren Oxidoreduktaseaktivität in den oxidierten Zustand NAD⁺ und/oder NADP⁺ überführt. Dabei bezeichnen NAD⁺ die oxidierte Form und NADH die reduzierte Form von Nicotinamidadenindinucleotid, während NADP⁺ die oxidierte Form und NADPH die reduzierte Form von Nicotinamidadenindinucleotidphosphat bezeichnen.

Zur Überführung reduzierter Kofaktoren in die oxidierte Form eignet sich als Oxidoreduktaseaktivität zum Beispiel eine Alkoholdehydrogenase, eine Xylose-Reduktase, eine Lactat-Dehydrogenase, eine Oxidase, Redoxenzyme, die an eine Elektrode gekoppelt werden, wie eine Alkoholdehydrogenase, eine Lactat-Dehydrogenase, eine Oxidase, Redoxenzyme, die an eine Elektrode gekoppelt werden.

Entsprechend ist das Verfahren gemäß vorliegender Erfindung dadurch gekennzeichnet, dass die von einer oder mehreren Oxidoreduktasen reduzierten Redoxkofaktoren NADH und/oder NADPH im selben Reaktionsansatz mittels einer Alkoholdehydrogenase, einer Lactat-Dehydrogenase, einer Xylose-Reduktase, einer

Oxidase, eines oder mehrerer Redoxenzyme, die an eine Elektrode gekoppelt werden, in den oxidierten Zustand NAD⁺ und/oder NADP⁺ überführt wird/werden.

Durch den Einsatz von einer oder mehreren Oxidoreduktaseaktivitäten zur Rücküberführung des/der reduzierten Redoxkofaktor(en) NADH und/oder NADPH in den oxidierten Zustand NAD⁺ und/oder NADP⁺ im selben Reaktionsansatz wird die Verwendung großer Mengen an kostenintensiven Redoxkofaktor(en) vermieden, sodass das Verfahren dadurch wirtschaftlich wird.

Ein weiterer Vorteil eines Verfahrens gemäß vorliegender Erfindung ist, dass das Hydrolysat aus der Hydrolyse der hemicellulosehaltigen Biomasse direkt zur Umwandlung der monomeren Zucker eingesetzt werden kann, ohne dass es einer Aufreinigung oder Aufkonzentrierung dieser bedarf. Die Konversion der monomeren Zucker kann direkt in einem Gemisch aus Zuckern, z.B. verschiedenen Zuckern, gegebenenfalls nicht hydrolysierten Zuckerpolymeren und weiterhin gegebenenfalls noch vorhandenen Feststoffen erfolgen. Ein weiterer Vorteil des Verfahrens ist, dass die monomeren Zucker durch die Umwandlung in Verbindungen, die zumindest eine ionische Bindungsstelle aufweisen, sehr einfach aus dem Hydrolysat isoliert und aufkonzentriert werden können. So können sie von den anderen Komponenten, die zum Beispiel nicht umgesetztes Xylan oder Xylooligosaccharide sein können, leicht abgetrennt werden. Durch die Wahl des entsprechenden Enzyms können auch spezifisch nur C5 Zucker oder nur C6 Zucker umgesetzt und abgetrennt werden, während alle anderen Zucker in der Lösung verbleiben. In einem Verfahren gemäß vorliegender Erfindung werden bevorzugt C5 Zucker umgesetzt.

In einem Verfahren gemäß vorliegender Erfindung kann die Konzentration entstandener Verbindungen, die eine ionische Bindungsstelle aufweisen, in der Mischung durch ein Trennverfahren gesenkt werden. Beispiele für solche Trennverfahren schließen Ionenaustauscherchromatographie, z.B. Anionenaustauscherchromatographie, und/oder Elektrodialyse ein.

In einem weiteren Aspekt stellt die vorliegende Erfindung ein Verfahren gemäß vorliegender Erfindung zur Verfügung, in dem entstandene Verbindungen, die eine ionische Bindungsstelle aufweisen, insbesondere mit Hilfe von Ionenaustauscherchromatographie und/oder Elektrodialyse aus der Reaktionsmischung abgetrennt werden.

Dadurch wird die Konzentration entstandener Verbindungen, die eine ionische Bindungsstelle aufweisen, in der Mischung gesenkt.

Eine solche Abtrennung kann an beliebiger Stelle eines Verfahrens gemäß vorliegender Erfindung erfolgen, sobald Verbindungen, die eine ionische Bindungsstelle aufweisen vorliegen.

Zucker, die in einem Verfahren gemäß vorliegender Erfindung nicht umgesetzt werden, können beispielsweise weiteren enzymatischen und/oder nicht enzymatischen Verfahren unterworfen werden.

In einem weiteren Aspekt stellt die vorliegende Erfindung das Verfahren gemäß vorliegender Erfindung zur Verfügung, dadurch gekennzeichnet, dass Arabonsäure, 2-Keto-3-deoxyarabonsäure, α-Ketoglutarsäure-Semialdehyd und/oder α-Ketoglutarsäure aus lignocellulosehaltigem Material hergestellt wird.

In den nachfolgenden Beispielen ist die Temperatur in Grad Celsisus (°C) angegeben.

### Beispiel 1

### Enzymatische Hydrolyse von Hemicellulose enthaltendem Material

Xylan wird in einer Konzentration von 8 % (w/v) in Acetat-Puffer mit pH 4,3 suspendiert und mit der ACCELLERASE® TRIO™ der Firma Genencor in einer Konzentration von 1 g Enzymlösung pro 1 g Xylan versetzt. Der Ansatz wird für 24 h bei 50°C gerührt. Der pH-Wert wird überprüft und im Falle einer Abweichung über 4,5 oder unter 4,1 nachjustiert. Der Ansatz wird über einen Büchner-Trichter filtriert und das Filtrat (Hydrolysat) wird mittels HPLC-LEX-DAD auf seine Zusammensetzung an Monomeren und deren Konzentration hin analysiert. Im Filtrat ist eine Konzentration von ca. 6 % Xylose, 0,43 % Arabinose und 0,27 % Glucose enthalten. Etwa 85 % der im Xylan erhaltenen Xylose wird so in monomerer Form erhalten.

### Beispiel 2

### Analytik des Hydrolysat mittels HPLC

500 µl des Filtrats der Xylan-Hydrolyse nach Beispiel 1 werden zentrifugiert und der Überstand dann über einen 0,2 µM PVDF (poly-vinyliden-difluorid) Filter gegeben und mittels HPLC-LEX-RID analysiert (Agilent Technologies Inc.). Aufgetrennt werden die Zucker dabei über eine Blei-Säule (Shodex® Sugar SP0810) von Shodex Denko K.K. mit einem Fluss von 0,5 ml/min Wasser (VWR: HPLC Grade) bei 80°C. Die Detektion erfolgt mittels Agilent RID. Es wird ein Inlinefilter von Agilent Technologies Inc., sowie als Vorsäulen eine Reversed-Phase Säule (Axpak-WA-G), eine Anionen-Austauscher-Säule (Shodex® Asahipak® ODP-50 6E) und eine Zucker-Vorsäule (Shodex® SP-G) jeweils von von Showa Denko K.K. verwendet.

### Beispiel 3

### Oxidation von L-Arabinose zu Arabonat durch eine Arabinose-Dehydrogenase mit Kofaktorrecycling über eine Alkohol-Dehydrogenase und anschließende Hydrolyse des Lactons durch Natronlauge

Ein 0,5 ml Ansatz enthält 50 mg/ml Arabinose, 5 U/ml der rekombinanten Arabinose-Dehydrogenase aus *Burkholderia vietnamiensis* und eine Mischung aus 0,5 mM NADP⁺ und 0,5 mM NADPH. Zur Regeneration des Kofaktors werden 2,5 % (w/v) Aceton und 5 U/ml der rekombinanten Alkoholdehydrogenase aus *Lactobacillus kefir* zugefügt. Die Enzyme werden in Form von Zelllysat eingesetzt. Die Reaktion findet für 24 h bei 40°C und pH 10 unter kontinuierlichem Schütteln (900 rpm) statt. Nach 24 h wird das Reaktionsgefäß bei 60°C für 10 min inkubiert, um die Enzyme zu inaktivieren. Anschließend werden 5 µl 2 M NaOH zugefügt.

So werden über 60 % der L-Arabinose in Natrium-L-Arabonat überführt. Die Analytik erfolgt mit GC-MS.

### Beispiel 4

### Oxidation von L-Arabinose zu Arabonat durch eine Arabinose-Dehydrogenase mit Kofaktorrecycling über eine Alkohol-Dehydrogenase und anschließende Hydrolyse des Lactons durch eine Lactonase

Ein 0,5 ml Ansatz enthält 50 mg/ml Arabinose, 5 U/ml der rekombinanten Arabinose-Dehydrogenase aus *Burkholderia vietnamiensis* und eine Mischung aus 0,5 mM NADP⁺ und 0,5 mM NADPH. Zur Regeneration des Kofaktors werden 2,5 % (v/v) Aceton und 5 U/ml der rekombinanten Alkoholdehydrogenase aus *Lactobacillus kefir* zugefügt. Die Enzyme werden in Form von Zelllysat eingesetzt. Die Reaktion findet für 24 h bei 40°C und pH 10 unter kontinuierlichem Schütteln (900 rpm) statt. Nach 24 h wird das Reaktionsgefäß bei 60°C für 10 min inkubiert, um die Enzyme zu inaktivieren. Nach dem Abkühlen werden 50µl eines *E.coli* Zelllysat mit überexprimierter L-Arabinolactonase aus *Azospirillum brasiliense* zugefügt und die Reaktion für weitere 24 h bei 40°C geschüttelt (900 rpm). Anschließend wird das Reaktionsgefäß bei 60°C für 10 min inkubiert, um das Enzym zu inaktivieren.
So werden über 65 % der L-Arabinose in L-Arabonat überführt. Die Analytik erfolgt mit GC-MS.

### Beispiel 5

### Analytik der Oxidationsreaktionen mittels GC-MS

Für die Analytik der Oxidationsreaktionen an der GC-MS müssen Substrate und Produkte derivatisiert werden. Die Ansätze werden zentrifugiert, über einen 0,2 µM PVDF-Filter gegeben und 1:30 verdünnt. Von der Verdünnung werden 20 µl in ein 0,5 ml Vial überführt und in der Speedvac getrocknet. Zum Derivatisieren werden dann 150 µl Pyridin und 50 µl einer 99:1-Mischung aus N,O-Bis(trimethylsilyl)trifluoroacetamid und Trimethylchlorosilan zugegeben. Im Pyridin ist als interner Standard Sorbitol in einer Konzentration von 0,1 mg/ml enthalten. Die Derivatisierung erfolgt für 16 h bei 60°C. Die Proben werden anschließend über GC-MS analysiert. Die Proben werden dabei über die Trennsäule HP-5ms (5 %-Phenyl)-methylpolysiloxan im Gaschromatograph aufgetrennt und dem Massenspektrometer GCMS QP210 Plus von Shimadzu analysiert.

### Beispiel 6

### Umwandlung von Arabinose zu Arabonat/Arabinolacton in einer Mischung aus Xylose und Arabinose

180 mg D-Xylose und 20 mg L-Arabinose wurden zusammen mit 2 U L-Arabinose-Dehydrogenase aus *Burkholderia vietnamiensis* sowie 2 U D-Xylose-Reduktase aus *Candida parapsilosis* in 50 mM wässrigem Tris-Puffer (pH = 7.0 bei 25°C) zu einem Gesamtvolumen von 500 µl gelöst. Die Reaktion fand in einem geschlossenen Reaktionsgefäß bei 40°C unter Schütteln (900 rpm, Eppendorf Thermomix®) statt. Nach 30 min wurden die Enzyme durch eine Inkubation bei 65°C für 15 min inaktiviert, denaturierte Proteine durch Zentrifugation (21000 g, 5 min) abgetrennt und die Zucker mittels GC-MS quantifiziert. Es wurde die eingesetzte L-Arabinose vollständig umgesetzt, 92 % davon zu L-Arabonat oder L-Arabino-γ-lacton und die restlichen 8 % zu L-Arabitol. Von der eingesetzten D-Xylose verblieben ca. 89,5 % während 10,4 % zu Xylitol umgesetzt wurden. Es wurde < 0,1 % der eingesetzten D-Xylose zu D-Xylonat/D-Xylono-γ-lacton oxidiert.

Der hier erreichte relativ selektive Umsatz von Arabinose zu Arabonat/Arabinolacton resultiert aus der höheren spezifischen Aktivität der Arabinose-Dehydrogenase für Arabinose im Vergleich zu Xylose, aus den relativen Anteilen von Arabinose und Xylose in der Reaktionsmischung sowie der limitierten Enzymaktivität/Reaktionszeit.

Das Beispiel zeigt unter anderem, dass man mit Hilfe eines Verfahrens gemäß vorliegender Erfindung spezifische Zucker aus einem Zuckergemisch abtrennen kann.

## Patentansprüche

1. Verfahren zur Gewinnung von Arabonsäure aus hemicellulosehaltigem Material, wobei
a) das hemicellulosehaltige Material enzymatisch oder nicht-enzymatisch hydrolysiert wird und das erhaltene Hydrolysat als freigesetzte Zucker Arabinose und Xylose enthält, und
b) das erhaltene Hydrolysat einer Umsetzung unterworfen wird, **gekennzeichnet durch** die Kombination der folgenden Maßgaben:
- die Arabinose wird mit Hilfe einer Oxidoreduktase in das γ-Arabinolacton übergeführt und γ-Arabinolacton zu Arabonsäure hydrolysiert, wobei von der Oxidoreduktase Redoxkofaktoren zu NADH und/oder NADPH reduziert werden, und
- die reduzierten Redoxkofaktoren NADH und/oder NADPH werden im selben Reaktionsansatz mittels einer Alkoholdehydrogenase, einer Lactat-Dehydrogenase, einer Xylose-Reduktase, einer Oxidase, und/oder eines oder mehrerer Redoxenzyme, die an eine Elektrode gekoppelt werden, in den oxidierten Zustand NAD⁺ und/oder NADP⁺ übergeführt.

2. Verfahren nach Anspruch 1, **dadurch gekennzeichnet, dass** die Arabonsäure zu 2-Keto-3-desoxyarabonsäure dehydratisiert wird.

3. Verfahren nach Anspruch 2, **dadurch gekennzeichnet, dass** die Arabonsäure mittels einer Dehydratase dehydratisiert wird.

4. Verfahren nach Anspruch 2 oder 3, **dadurch gekennzeichnet, dass** die 2-Keto-3-desoxyarabonsäure zum α-Ketoglutarsäure-Semialdehyd weiter dehydratisiert wird.

5. Verfahren nach Anspruch 4, **dadurch gekennzeichnet, dass** die 2-Keto-3-desoxyarabonsäure mittels einer Dehydratase weiter dehydratisiert wird.

6. Verfahren nach Anspruch 4 oder 5, **dadurch gekennzeichnet, dass** der α-Ketoglutarsäure-Semialdehyd zu α-Ketoglutarsäure oxidiert wird.

7. Verfahren nach Anspruch 6, **dadurch gekennzeichnet, dass** der α-Ketoglutarsäure-Semialdehyd mittels einer Oxidoreduktase oxidiert wird.

8. Verfahren nach einem der Ansprüche 1 bis 7, **dadurch gekennzeichnet, dass** entstandene Verbindungen, die eine ionische Bindungsstelle aufweisen abgetrennt werden.

9. Verfahren nach einem der Ansprüche 1 bis 8, **dadurch gekennzeichnet, dass** vor der Umsetzung gemäß Anspruch 1 das hemicellulosehaltige Material aus lignocellulosischem Material hergestellt wird.

10. Verfahren nach Anspruch 9, **dadurch gekennzeichnet, dass** das hemicellulosehaltige Material durch Behandlung von lignocellulosischem Material mit einer alkalischen, wässrigen Alkohollösung hergestellt wird.

11. Verfahren nach einem der Ansprüche 9 oder 10, **dadurch gekennzeichnet, dass** das lignocellulosehaltige Material lignocellulosehaltige Biomasse ist.

12. Verfahren nach einem der Ansprüche 1 bis 11, **dadurch gekennzeichnet, dass** die freigesetzten Zucker eine Mischung aus L-Arabinose und D-Xylose darstellen.

13. Verfahren nach einem der Ansprüche 8 bis 12, **dadurch gekennzeichnet, dass** entstandene Verbindungen mit Hilfe von Ionenaustauscherchromatographie und/oder Elektrodialyse aus der Reaktionsmischung abgetrennt werden.

14. Verfahren nach einem der Ansprüche 11 bis 13, **dadurch gekennzeichnet, dass** das lignocellulosehaltige Material aus Einjahrespflanzen, Stroh, Energiegräsern, Sisal, Bagasse, oder untypischen Lignocellulosesubstraten, wie Spelzen, ist.

15. Verfahren nach einem der Ansprüche 9 bis 14, **dadurch gekennzeichnet, dass** Arabonsäure, 2-Keto-3-deoxyarabonsäure, α-Ketoglutarsäure-Semialdehyd und/oder α-Ketoglutarsäure aus lignocellulosehaltigem Material hergestellt wird.

## Claims

1. A process of obtaining arabonic acid from a hemicellulose-containing material, wherein
a) the hemicellulose-containing material is hydrolyzed enzymatically or non-enzymatically and the obtained hydrolysate includes arabinose and xylose as released sugars, and
b) the obtained hydrolysate is subjected to conversion, **characterized by** a combination of the following provisos:
- the arabinose is converted into the γ-arabinolactone by means of an oxidoreductase and γ-arabinolactone is hydrolyzed into arabonic acid, wherein redox cofactors are reduced to NADH and/or NADPH by the oxidoreductase, and
- the reduced redox cofactors NADH and/or NADPH are converted into the oxidized state NAD⁺ and/or NADP⁺ in the same reaction batch by means of an alcohol dehydrogenase, a lactate dehydrogenase, a xylose reductase, an oxidase and/or one or several redox enzymes coupled to an electrode.

2. A process according to claim 1, **characterized in that** the arabonic acid is dehydrated into 2-keto-3-deoxyarabonic acid.

3. A process according to claim 2, **characterized in that** the arabonic acid is dehydrated by means of a dehydratase.

4. A process according to claim 2 or 3, **characterized in that** the 2-keto-3-deoxyarabonic acid is dehydrated further into α-ketoglutaric acid semialdehyde.

5. A process according to claim 4, **characterized in that** the 2-keto-3-deoxyarabonic acid is dehydrated further by means of a dehydratase.

6. A process according to claim 4 or 5, **characterized in that** the α-ketoglutaric acid semialdehyde is oxidized to α-ketoglutaric acid.

7. A process according to claim 6, **characterized in that** the α-ketoglutaric acid semialdehyde is oxidized by means of an oxidoreductase.

8. A process according to any of claims 1 to 7, **characterized in that** resulting compounds exhibiting an ionic binding site are separated.

9. A process according to any of claims 1 to 8, **characterized in that**, prior to the conversion according to claim 1, the hemicellulose-containing material is produced from a lignocellulosic material.

10. A process according to claim 9, **characterized in that** the hemicellulose-containing material is produced by treating a lignocellulosic material with an alkaline, aqueous alcohol solution.

11. A process according to any of claims 9 to 10, **characterized in that** the lignocellulose-containing material is a lignocellulose-containing biomass.

12. A process according to any of claims 1 to 11, **characterized in that** the released sugars constitute a mixture of L-arabinose and D-xylose.

13. A process according to any of claims 8 to 12, **characterized in that** resulting compounds are separated from the reaction mixture by means of ion exchange chromatography and/or electrodialysis.

14. A process according to any of claims 11 to 13, **characterized in that** the lignocellulose-containing material is derived from annual plants, straw, energy grasses, sisal, bagasse, or atypical lignocellulose substrates such as husks.

15. A process according to any of claims 9 to 14, **characterized in that** arabonic acid, 2-keto-3-deoxyarabonic acid, α-ketoglutaric acid semialdehyde and/or α-ketoglutaric acid is/are produced from a lignocellulose-containing material.

## Revendications

1. Procédé de préparation d'acide arabonique à partir d'un matériau contenant de l'hémicellulose, dans lequel
a) on hydrolyse le matériau contenant de l'hémicellulose de façon enzymatique ou non enzymatique et le produit d'hydrolyse obtenu contient de l'arabinose et du xylose en tant que sucres libérés, et
b) on soumet le produit d'hydrolyse obtenu à une conversion,
**caractérisé par** la combinaison des conditions suivantes :
on transforme l'arabinose en gamma-arabinolactone à l'aide d'une oxydoréductase et
on hydrolyse la gamma-arabinolactone en acide arabonique, dans lequel des cofacteurs redox sont réduits par l'oxydoréductase en NADH et/ou en NADPH, et les cofacteurs redox réduits NADH et/ou NADPH sont transformés, dans le même effet réactif, en l'état oxydé NAD⁺ et/ou NADP⁺, au moyen d'une alcool déshydrogénase, d'une lactate déshydrogénase, d'une oxydase, et/ou d'une ou de plusieurs enzyme(s) redox, qui est/sont couplée(s) à une électrode.

2. Procédé selon la revendication 1, **caractérisé en ce que** l'on déshydrate l'acide arabonique en acide 2-céto-3-désoxy arabonique.

3. Procédé selon la revendication 2, **caractérisé en ce que** l'on déshydrate l'acide arabonique au moyen d'une déshydratase.

4. Procédé selon la revendication 2 ou 3, **caractérisé en ce que** l'on déshydrate encore l'acide 2-céto-3-désoxy arabonique en semi-aldéhyde de l'acide alpha-cétoglutarique.

5. Procédé selon la revendication 4, **caractérisé en ce que** l'on déshydrate encore l'acide 2-céto-3-désoxy arabonique au moyen d'une déshydratase.

6. Procédé selon la revendication 4 ou 5, **caractérisé en ce que** l'on oxyde le semi-aldéhyde de l'acide alpha-cétoglutarique en acide alpha-cétoglutarique.

7. Procédé selon la revendication 6, **caractérisé en ce que** l'on oxyde le semi-aldéhyde de l'acide alpha-cétoglutarique au moyen d'une oxydoréductase.

8. Procédé selon l'une quelconque des revendications 1 à 7, **caractérisé en ce que** l'on sépare des composés produits, qui présentent un point de liaison ionique.

9. Procédé selon l'une quelconque des revendications 1 à 8, **caractérisé en ce que** l'on fabrique, avant la conversion selon la revendication 1, le matériau contenant de l'hémicellulose à partir de matériau lignocellulosique.

10. Procédé selon la revendication 9, **caractérisé en ce que** l'on fabrique le matériau contenant de l'hémicellulose par traitement de matériau lignocellulosique avec une solution alcaline aqueuse d'alcool.

11. Procédé selon une des revendications 9 ou 10, **caractérisé en ce que** le matériau contenant de la lignocellulose est une biomasse contenant de la lignocellulose.

12. Procédé selon l'une quelconque des revendications 1 à 11, **caractérisé en ce que** les sucres libérés représentent un mélange de L-arabinose et de D-xylose.

13. Procédé selon l'une quelconque des revendications 8 à 12, **caractérisé en ce que** l'on sépare des composés produits hors du mélange de réaction à l'aide de la chromatographie sur échangeurs d'ions et/ou à l'aide d'une électrodialyse.

14. Procédé selon l'une quelconque des revendications 11 à 13, **caractérisé en ce que** le matériau contenant de la lignocellulose est constitué par des plantes annuelles, de la paille, des herbes énergétiques, du sisal, de la bagasse, ou des substrats lignocellulosiques non typiques, tels que des balles de végétaux.

15. Procédé selon l'une quelconque des revendications 9 à 14, **caractérisé en ce que** l'on fabrique de l'acide arabonique, de l'acide 2-céto-3-désoxy arabonique, du semi-aldéhyde de l'acide alpha-cétoglutarique et/ou de l'acide alpha-cétoglutarique à partir de matériau contenant de la lignocellulose.
